(19) **Europäisches Patentamt / European Patent Office / Office européen des brevets**

(11) **EP 2 992 905 A1**

(12) **EUROPEAN PATENT APPLICATION**

(43) Date of publication:
09.03.2016 Bulletin 2016/10

(51) Int Cl.:
*A61K 51/08* [(2006.01)] *A61K 51/12* [(2006.01)]
*A61P 35/00* [(2006.01)]

(21) Application number: 14192599.0

(22) Date of filing: 11.11.2014

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(30) Priority: 02.09.2014 TW 103130273

(71) Applicant: **Institute of Nuclear Energy Research Atomic Energy Council**
**Longtan Township, Taoyuan County (TW)**

(72) Inventors:
• **Chen, Su-Jung**
**Taoyuan County (TW)**

• **Li, Chung-Yen**
**Taoyuan County (TW)**
• **Lee, Te-Wei**
**114 Taipei City (TW)**
• **Chang, Chih-Hsien**
**300 Hsinchu City (TW)**

(74) Representative: **Lang, Christian**
**LangPatent Anwaltskanzlei**
**IP Law Firm**
**Rosenheimer Straße 139**
**81671 München (DE)**

(54) **Kit and method for quickly preparing radio-isotope labeled human serum albumin microspheres**

(57)   The present disclosure relates to a kit for preparing radio-isotope labeled human serum albumin (HSA) microspheres, which includes: a container (A), containing $SnCl_2$ dissolved in an aqueous acid solution; a container (B), containing an acidic substance as a tin salt stabilizer; a container (C), containing HSA microspheres to be labeled by a radio-isotope; and a container (D), containing a pH adjuster. According to the present kit for quickly preparing radio-isotope labeled HSA microspheres, HSA microspheres can be simply and quickly labeled by a radio-isotope at high labeling efficiency.

The present disclosure also relates to a method for quickly preparing radio-isotope labeled HSA microspheres by using the kit.

FIG. 1

Printed by Jouve, 75001 PARIS (FR)

## Description

### TECHNICAL FIELD

The present disclosure relates to a kit for quickly preparing radio-isotope labeled human serum albumin (HSA) microspheres and a method for quickly preparing radio-isotope labeled HSA microspheres by using the kit, which can be applied to angiographic diagnosis and treatment of tumors by simply and quickly labeling a radio-isotope on HSA microspheres at high labeling efficiency.

### BACKGROUND

As a drug for in vivo radiotherapy of tumors, radio-isotope labeled microspheres have been widely used in tumor treatment, and the main treatment manner is directly guiding radioactive microspheres to a tumor site by using a duct. Since radioactive microspheres have a particle diameter greater than the microvascular diameter, the microvessels of the tumor are closed, so the supply of nutrient to the tumor is blocked, so necrosis of the tumor is accelerated. Moreover, the radioactive microspheres may also be concentrated at the tumor site to selectively increase the radioactivity to directly give damage to tumor cells, which can reduce the damage to other normal cells from the radioactive drug. In order to achieve therapeutic effects, many radio-isotopes such as yttrium-90 ($^{90}$Y), rhenium-188 ($^{188}$Re), rhenium-186 ($^{186}$Re), technetium-99m ($^{99m}$Tc) and holmium-166 ($^{166}$Ho) have been studied as nuclides for radiotherapy and are labeled on microspheres. The isotope rhenium-188 can release β-rays, has a maximum energy of 2.12 MeV and a half-life of 16.9 hr, and is suitable for being used in treatment; and at the same time, the isotope rhenium-188 can release γ-rays of 155 KeV, and is suitable for being used in angiographic diagnosis. This nuclide is generated by a tungsten-188/rhenium-188 generator, which is convenient to use in hospitals.

Microspheres synthesized with HSA microspheres as raw material are ideal radio-isotope supports and have the following advantages: (1) being biodegradable and biocompatible: different from other microspheres synthesized with glass or plastics as raw material, the HSA microspheres is biodegradable and has no antigenicity, thus avoiding the security risk of permanently remaining in the human body; (2) being capable of performing labeling reaction in a high-temperature environment, thus facilitating radionuclide labeling; (3) having high drug stability after labeling; and (4) using microspheres in a specific size range, being capable of maintaining stable structure and configuration in a high-temperature environment, and being suitable for radionuclide labeling.

In 2005, Wunderlich set forth a method for labeling HSA microspheres with rhenium-188, where according to the labeling process, rhenium-188 is reduced by stannous chloride, and reduced rhenium-188 is adsorbed and precipitate on the surface of the microspheres (US20080219923 A1). Although this method significantly improves the labeling efficiency and reduces the amount of stannous chloride, the reaction needs to be performed in a water bath of about 90°C, and 90% labeling efficiency can be achieved after a reaction time of 45 to 70 min, while for synthesis of drugs labeled by a radio-isotope having a short half-life, the activity loss of the radio-isotope needs to be taken into consideration, so the long period of reaction time is not conducive to commercial applications. Moreover, it is found that the stability in serum is reduced with time, and after 48 hr at room temperature, the stability is reduced to 86% of the original value. Upon in vivo applications, due to the reduced stability, the efficacy is influenced, and free rhenium-188 on the surface of the microspheres may be distributed on other parts of the organism, thus causing risks of toxicity.

### SUMMARY

The present invention is completed in view of the above situation, and objectives of the present invention are to provide a kit and a method for simply and quickly preparing radio-isotope labeled HSA microspheres by labeling a radio-isotope on HSA microspheres at high labeling efficiency.

The present invention provides a kit for quickly preparing radio-isotope labeled HSA microspheres, which comprises: a container (A), containing $SnCl_2$ dissolved in aqueous acid solution; a container (B), containing a tin salt stabilizer used for radio-isotope stabilization; a container (C), containing HSA microspheres having a particle diameter in the range of 10 to 60 μm and to be labeled by a radio-isotope; a container (D), containing a pH adjuster for adjusting the pH value suitable for administration to human body such as a pH value in the range of 6 to 8 after bonding and a reduction reaction of the reagents in the containers (A) to (C) and the radio-isotope nuclide are completed.

According to the kit for quickly preparing radio-isotope labeled HSA microspheres of the present invention, the aqueous acid solution in the container (A) is at least one selected from aqueous hydrochloric acid, phosphoric acid and acetic acid solution.

According to the kit for quickly preparing radio-isotope labeled HSA microspheres of the present invention, the tin salt stabilizer in the container (B) is at least one selected from citric acid, oxalic acid, gallic acid, salicylic acid, tartaric acid, gluconic acid, ascorbic acid and benzoic acid.

According to the kit for preparing radio-isotope labeled HSA microspheres of the present invention, the particle diameter of the HSA microspheres in the container (C) is in the range of 10 to 60 $\mu$m, and the HSA microspheres may be HSA microspheres prepared by a conventional method and may also be commercially available HSA microspheres, for example, model ROTOP-HSA B20 purchased from Rotop Company (Germany), containing 2.5 mg HSA microspheres per dose and containing 300,000 to 500,000 microspheres having a particle diameter of about 10 to 30 $\mu$m, but not limited thereto, provided that HSA microspheres having a particle diameter in the range of 10 to 60 $\mu$m can be used.

According to the kit for preparing radio-isotope labeled HSA microspheres of the present invention, the pH adjuster in the container (D) is not particularly limited and may be a basic compound that can adjust the pH value of the solution finally obtained after mixing the reagents in containers (A) to (C) to the range described above, and includes, for example, NaOH, ammonia, Tris buffer, PBS buffer and phosphate, with an aqueous NaOH solution being preferred. The concentration and amount of the pH adjuster are also not particularly limited, provided that the pH value of the solution finally obtained after mixing the reagents in containers (A) to (C) is adjusted to the range described above.

According to the kit for preparing radio-isotope labeled HSA microspheres of the present invention, for the convenience in use, the kit dose may be designed to be a single dose after mixing, so that merely one kit is needed to complete the mixing of the required reagents upon each time of use. At this time, the contents of the reagents in the containers are respectively as follows, the mass of $SnCl_2$ in the container (A) is in the range of 2.5 mg to 10 mg, and preferably in the range of 3.5 mg to 4.5 mg, the mass of the tin salt stabilizer in the container (B) is in the range of 10 mg to 30 mg, and preferably in the range of 15 mg to 25 mg, and the mass of the HSA microspheres in the container (C) is in the range of 2.0 mg to 3.0 mg. According to the specification of the designed dose, the amount of the radio-isotope for labeling the HSA microspheres is 10 to 200 mCi/mL by specific activity.

The nuclide to be labeled by a radio-isotope by using the kit of the present invention may be rhenium-188 ($^{188}$Re), rhenium-186 ($^{186}$Re), technetium-99m ($^{99m}$Tc), and includes, for example, sodium perrhenate ($^{188}$ReO$_4$Na or $^{186}$ReO$_4$Na) or sodium technetate ($^{99m}$TcO$_4$Na) (which can generate perrhenate radical ($^{188}$ReO$_4{}^-$ or $^{186}$ReO$_4{}^-$) or technetate radical ($^{99m}$TcO$_4{}^-$) in an aqueous solution through disassociation, which can be reduced into radio-isotope rhenium-188 ($^{188}$Re) or rhenium-186 ($^{186}$Re) or technetium-99m ($^{99m}$Tc) from a high oxidation number to a low oxidation number by $SnCl_2$.

A method for preparing radio-isotope labeled HSA microspheres, which is characterized by using the kit for labeling HSA microspheres with a radio-isotope comprising containers (A) to (D), and comprising steps of:

(1) respectively injecting a saline solution into a container (A) and a container (B) to form an aqueous solution, next, respectively injecting the solution in the container (A) and the solution in the container (B) to a container (C), and then, injecting a radio-isotope into the container (C) and fully mixing it with the solution;

(2) placing the container (C) in the step (1) into a microwave reactor for a labeling reaction at a microwave power of 40 to 200 W for a reaction time of 1 to 10 min, and at the same time, reducing the radio-isotope from a high oxidation number to a low oxidation number, and bonding the radio-isotope on the HSA microspheres, to obtain radio-isotope labeled HSA microspheres,

where, according to the method for preparing radio-isotope labeled HSA microspheres of the present invention, the radio-isotope may be rhenium-188 ($^{188}$Re), rhenium-186 ($^{186}$Re) or technetium-99m ($^{99m}$Tc); and

(3) adding a pH adjuster in a container (D) to the radio-isotope labeled HSA microspheres obtained in Step (2) to adjust the pH value to a pH value in the range of 6 to 8.

According to the method for preparing radio-isotope labeled HSA microspheres of the present invention, since the radio-isotope easily decays with time, before starting the method of the present invention elutriation of the radio-isotope nuclide is performed. A generator for manufacturing the radio-isotope nuclide is not particularly limited, but, for example, the $^{188}$W/$^{188}$Re generator manufactured by the National Institute for Radioelement (IRE, Belgium) can be used for elutriation.

The method for preparing radio-isotope labeled HSA microspheres of the present invention has the following advantages: (1) being easy to operate with high labeling efficiency, so that the reaction time can be reduced, thus being suitable for a radio-isotope that decays with time, especially a radio-isotope having a short half-life, achieving the benefit of low cost, and increasing the useful life of radioactive drugs due to the reduced reaction time; (2) being applicable in treatment of hepatic artery embolization microspheres due to the particle diameter in the range of 10 to 60 $\mu$m and ingredients being easily degraded (3) having good drug stability; and (4) having double efficacies of radiodiagnosis and cancer treatment.

## BRIEF DESCRIPTION OF DRAWINGS

FIG. 1 is a flowchart of implementation of a method for preparing radio-isotope labeled HSA microspheres of the present invention; and

FIG. 2 is chart of analysis results of stability over time of $^{188}$Re-HSA microspheres according to Example 3.

## DETAILED DESCRIPTION OF DISCLOSED EXAMPLES

In the following, the present invention is further described with a Synthetic Example and Examples, but the Synthetic Example and Examples are merely used to illustrate the present invention, but not intended to limit the scope of the present invention. For example, in the following Examples, merely rhenium-188 radio-isotope is used as an example for illustration, but the present invention is not limited thereto, and rhenium-186 ($^{186}$Re) or technetium-99m ($^{99m}$Tc) may also be used as the radio-isotope.

[Synthesis Example] Synthesis of HSA microspheres

Step 1: To a 1,000 mL breaker, 800 mL olive oil was added, and then the breaker was placed on a temperature-controlled heater equipped with a magnet heating mixer, and heated to a temperature of 60°C for pre-heating for at least 30 min at a rotation rate of 520 rpm. In the temperature-controlled heater, a micro-motor drives a high temperature-resistant powerful magnet to rotate and generate a rotating magnetic field, so that stirrers in a container are driven to rotate, and at the same time, the solution in the container is heated simultaneously, so as to allow the solution to be fully mixed and reacted.

Step 2: HSA (purchased from Sigma Company) was placed in 5 mL water to formulate an HSA solution having a concentration of 20% (w/v), and the HSA solution was drawn by a pump and added drop-wise into the breaker at an initial rate of 4 mL/min, and then the rate was gradually increased to 9 mL/min till addition was finished, and then the mixture was stirred for 3 min at a rotation rate of 520 rpm.

Step 3: The rotation rate of the temperature-controlled heater was adjusted to 240 rpm and the reaction was performed for 30 min, then the reaction temperature was adjusted to 110°C, and the reaction was performed for 30 min at a rotation rate of 50 rpm.

Step 4: Olive oil was removed after reaction by a 20 μm filter, oil molecules on the surface of the HSA microspheres was washed by acetone, and the resulting product was dried for a certain period of time at 40°C, and HSA microspheres having a particle diameter in the range of 10 to 60 μm were sieved out by a 60 μm screen and a 10 μm screen.

[Example 1]

Preparation of HSA microsphere kit

4 mg SnCl$_2$ and 400 uL 0.1N HCl were fully mixed and dissolved, nitrogen was charged into the container for about 1 min, after freeze-drying, and the container cap was sealed with a plastic cork and an aluminum cap, to obtain a container (A). Additionally, 20 mg citric acid was added into a container (B), nitrogen was charged into the container for about 1 min, and the container cap was sealed with a plastic cork and an aluminum cap. Additionally, 2.5 mg HSA microspheres prepared in Synthetic Example were placed in a container (C), nitrogen was charged into the container for about 1 min, and the container cap was sealed with a plastic cork and an aluminum cap. Additionally, 1 mL IN NaOH was placed in a container (D), and the container cap was sealed with a plastic cork and an aluminum cap.

[Example 2]

Preparation and analysis of rhenium-188 labeled HSA microspheres

1. 400 uL saline solution was added to a container (A) and fully mixed, to obtain a mixture (A). 400 uL saline solution was added to a container (B) and fully mixed, to obtain a mixture (B).

2. The mixture (A) and the mixture (B) obtained in Step 1 were extracted and injected into a container (C), and 600

μL radio-isotope rhenium-188 solution was injected to the container (C), where the activity of rhenium-188 was 25 mCi/mL.

3. The mixture obtained in Step 2 was transferred into a microwave reactor for a labeling reaction for 3 min at a microwave power of 90 W, to obtain a rhenium-188 labeled HSA microspheres (referred to as $^{188}$Re-HSA microspheres thereafter)-containing mixture.

4. Next, 300 μL solution in the container (D) was drawn and injected into the $^{188}$Re-HSA microspheres-containing mixture obtained in Step 3, to adjust the pH value to be 7.0.

5. Then, labeling efficiency measurement and particle diameter analysis were performed, the $^{188}$Re-HSA microspheres-containing mixture obtained in Step 4 was centrifuged for 5 min at 13,000 rpm, and the supernatant and precipitate were respectively taken for radioactivity measurements, and the labeling efficiency of the $^{188}$Re-HSA microspheres was calculated to be 97% according to the following formula for labeling efficiency.

$$\text{Labeling efficiency } (\%) = [(\text{precipitate activity})/(\text{supernatant activity} + \text{precipitate activity})] \times 100\%.$$

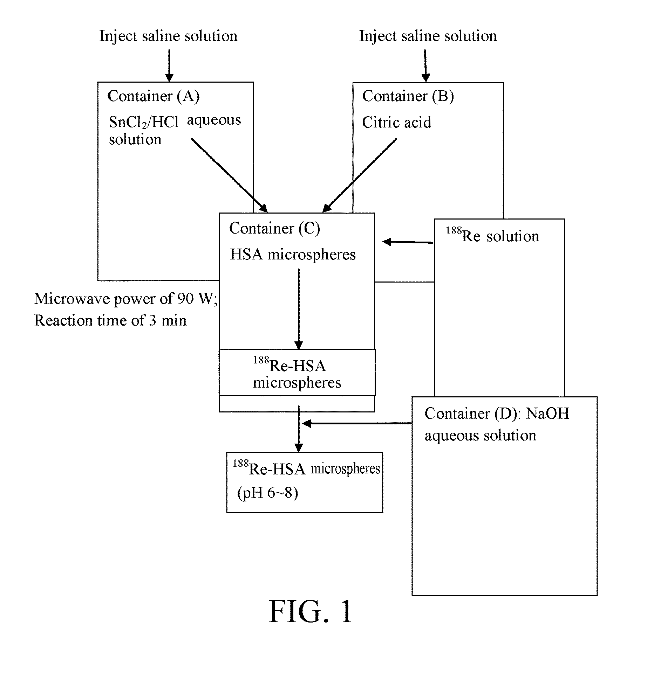

The particle diameter of the $^{188}$Re-HSA microspheres was measured by using a micron particle size analyzer, and the average particle diameter of the $^{188}$Re-HSA microspheres was measured to be about 24 μm.
The flowchart of Steps 1 to 4 is shown in FIG 1.

[Example 3] Analysis of stability over time of $^{188}$Re-HSA microspheres

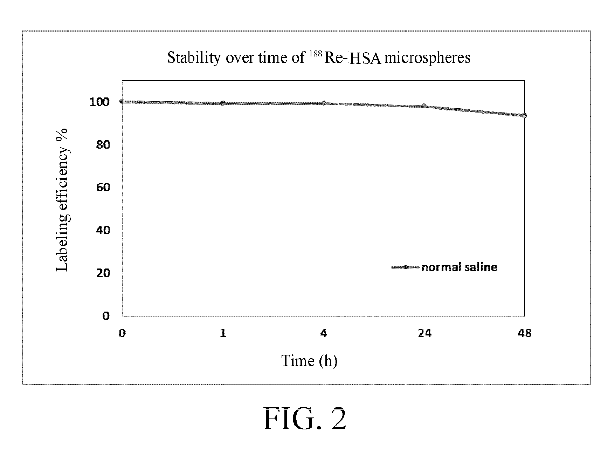

At room temperature, the $^{188}$Re-HSA microspheres-containing mixture obtained in Example 2 was placed in a 3000 μL saline solution, a 500 μL mixture was drawn respectively at 1 hr, 4 hr, 24 hr and 48 hr for labeling an efficiency measurement by the method in Step 5 of Example 2. It can be known from the test results that the $^{188}$Re-HSA microspheres still has a labeling efficiency greater than 90% after 48 hr in the saline solution, indicating that the $^{188}$Re-HSA microspheres prepared by the method of the present invention are considerably stable. The results are shown in FIG. 2 and Table 1.

Table 1 Results of stability over time

| Time (hr) | Microspheres bonded Radioactivity, Mean ±SD |
|---|---|
| | in saline solution, 25°C |
| 1 | 99.30±0.16 |
| 4 | 99.20±0.22 |
| 24 | 97.93±0.67 |
| 48 | 93.53±0.70 |
| Mean ± Standard deviation (SD), n=3 | |

It can be known from the above that, the method for quickly preparing radio-isotope labeled HSA microspheres of the present invention has the following advantages: (1) being easy to operate with high labeling efficiency, so that the reaction time can be reduced, thus being suitable for a radio-isotope that decays with time, especially a radio-isotope having a short half-life, achieving the benefit of low cost, and increasing the useful life of radioactive drugs due to the reduced reaction time; (2) being applicable in treatment of hepatic artery embolization microspheres due to the particle diameter in the range of 10 to 60 μm and ingredients being easily degraded (3) having good drug stability; and (4) having double efficacies of radiodiagnosis and cancer treatment.

**Claims**

1. A kit for quickly preparing radio-isotope labeled human serum albumin (HSA) microspheres, comprising: a container (A), containing SnCl$_2$ dissolved in an aqueous acid solution; a container (B), containing a tin salt stabilizer; a container (C), containing HSA microspheres to be labeled by an isotope; and a container (D), containing a pH adjuster.

2. The kit according to claim 1, wherein the aqueous acid solution in the container (A) is at least one of hydrochloric acid, phosphoric acid and acetic acid.

3. The kit according to claim 1, wherein the tin salt stabilizer in the container (B) is at least one selected from citric acid, oxalic acid, gallic acid, salicylic acid, tartaric acid, gluconic acid, ascorbic acid and benzoic acid.

4. The kit according to claim 1, wherein the particle diameter of the HSA microspheres in the container (C) is in the range of 10 to 60 $\mu$m.

5. The kit according to claim 1, wherein as for a single dose after mixing as designed, the contents of the reagents in the containers are respectively as follows, the mass of $SnCl_2$ in the container (A) is in the range of 2.5 mg to 10 mg, the mass of the tin salt stabilizer in the container (B) is in the range of 10 mg to 30 mg, and the mass of the HSA microspheres in the container (C) is in the range of 2.0 mg to 3.0 mg.

6. The kit according to claim 1, wherein the pH adjuster in the container (D) is at least one selected from NaOH, ammonia, Tris buffer, PBS buffer and phosphate.

7. A method for quickly preparing radio-isotope labeled human serum albumin (HSA) microspheres, which is **characterized by** using the kit according to any one of claims 1 to 6, and comprising steps of:

   (1) respectively injecting a saline solution into a container (A) and a container (B), next, respectively injecting the solution in the container (A) and the solution in the container (B) into a container (C), and then, injecting a radionuclide solution into the container (C);
   (2) placing the container (C) in step (1) into a microwave reactor for a labeling reaction at a specific microwave power for an appropriate reaction time; and
   (3) adding the reactant to a container (D) to adjust to an appropriate pH value, to obtain radio-isotope labeled HSA microspheres.

8. The method according to claim 7, wherein the radionuclide in the radionuclide solution in Step (1) is at least one of rhenium-188 ($^{188}$Re) or rhenium-186 ($^{186}$Re) and technetium-99m ($^{99m}$Tc).

9. The method according to claim 7, wherein the specific microwave power in Step (2) is 40 to 200 W.

10. The method according to claim 7, wherein the appropriate reaction time in Step (2) is 1 to 10 min.

11. The method according to claim 7, wherein the appropriate pH value in Step (3) is in the range of 6 to 8.

Inject saline solution        Inject saline solution

Container (A)

$SnCl_2$/HCl aqueous
solution

Container (B)

Citric acid

Container (C)

HSA microspheres

$^{188}Re$ solution

Microwave power of 90 W;
Reaction time of 3 min

$^{188}Re$-HSA
microspheres

Container (D): NaOH
aqueous solution

$^{188}Re$-HSA microspheres
(pH 6~8)

# FIG. 1

FIG. 2

Europäisches
Patentamt
European
Patent Office
Office européen
des brevets

# EUROPEAN SEARCH REPORT

Application Number

EP 14 19 2599

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | BORZA V N ET AL: "Labelling of albumin microspheres with < 188 > Re: A possible agent for radiotherapy", INTERNATIONAL JOURNAL OF NANO AND BIOMATERIALS, INDERSCIENCE PUBLISHERS, GB, vol. 2, no. 6, 1 September 2009 (2009-09-01), pages 540-546, XP008161025, ISSN: 1752-8933, DOI: 10.1504/IJNBM.2009.028342 [retrieved on 2009-09-17] * page 543 * | 1-11 | INV. A61K51/08 A61K51/12 A61P35/00 |
| X | WUNDERLICH G ET AL: "A kit for labeling of [<188>Re] human serum albumin microspheres for therapeutic use in nuclear medicine", APPLIED RADIATION AND ISOTOPES, ELSEVIER, OXFORD, GB, vol. 62, no. 6, 1 June 2005 (2005-06-01), pages 915-918, XP027715969, ISSN: 0969-8043 [retrieved on 2005-06-01] | 1-6 | |
| Y | * Abstract; pages 916-917 * | 7-11 | TECHNICAL FIELDS SEARCHED (IPC)<br><br>A61K |
| Y | D.-R. HWANG ET AL: "Application of microwave technology to the synthesis of short-lived radiopharmaceuticals", JOURNAL OF THE CHEMICAL SOCIETY, CHEMICAL COMMUNICATIONS., no. 23, 1 January 1987 (1987-01-01), page 1799, XP55239748, GB ISSN: 0022-4936, DOI: 10.1039/c39870001799 * abstract * | 1-11 | |

-/--

The present search report has been drawn up for all claims

| Place of search | Date of completion of the search | Examiner |
|---|---|---|
| The Hague | 12 January 2016 | Bettio, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 14 19 2599

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 2 609 936 A1 (INER AEC EXECUTIVE YUAN [TW]) 3 July 2013 (2013-07-03) * paragraphs 25-26 * ----- | 1-11 | |
| Y | CH 644 019 A5 (NEW ENGLAND NUCLEAR CORP [US]) 13 July 1984 (1984-07-13) * page 9, left-hand column * ----- | 7-11 | |
| A | US 2008/219923 A1 (WUNDERLICH GERD [DE] ET AL) 11 September 2008 (2008-09-11) * Paragraphs 62-65 * ----- | 1-11 | |

TECHNICAL FIELDS
SEARCHED     (IPC)

The present search report has been drawn up for all claims

| Place of search | Date of completion of the search | Examiner |
|---|---|---|
| The Hague | 12 January 2016 | Bettio, Andrea |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 14 19 2599

This annex lists the patent family members relating to the patent documents cited in the above-mentioned European search report.
The members are as contained in the European Patent Office EDP file on
The European Patent Office is in no way liable for these particulars which are merely given for the purpose of information.

12-01-2016

| Patent document cited in search report | | Publication date | Patent family member(s) | | Publication date |
|---|---|---|---|---|---|
| EP 2609936 | A1 | 03-07-2013 | EP | 2609936 A1 | 03-07-2013 |
| | | | TW | 201325624 A | 01-07-2013 |
| | | | US | 2013172532 A1 | 04-07-2013 |
| CH 644019 | A5 | 13-07-1984 | CA | 1112162 A | 10-11-1981 |
| | | | CH | 644019 A5 | 13-07-1984 |
| US 2008219923 | A1 | 11-09-2008 | AT | 370750 T | 15-09-2007 |
| | | | AU | 2005209037 A1 | 11-08-2005 |
| | | | CA | 2553235 A1 | 11-08-2005 |
| | | | CN | 1913926 A | 14-02-2007 |
| | | | DE | 102004005280 A1 | 01-09-2005 |
| | | | EP | 1713516 A2 | 25-10-2006 |
| | | | JP | 2007523062 A | 16-08-2007 |
| | | | KR | 20060131862 A | 20-12-2006 |
| | | | US | 2008219923 A1 | 11-09-2008 |
| | | | WO | 2005072781 A2 | 11-08-2005 |

EPO FORM P0459

For more details about this annex : see Official Journal of the European Patent Office, No. 12/82

**EP 2 992 905 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080219923 A1